# EUROPÄISCHE PATENTANMELDUNG

(11) **EP 1 129 704 A1**
(43) Veröffentlichungstag der Anmeldung: **05.09.2001**
(21) Anmeldenummer: 00104926.1
(22) Anmeldetag: 08.03.2000
(51) Int. Cl.: A61K 9/00

(54) **Verfahren zur selektiven Verabreichung zentralnervös aktiver Stoffe**

(30) Priorität: 02.02.2000 DE 10004547
(71) Anmelder: pharmed Holding GmbH, 82031 Grünwald b. München (DE)
(72) Erfinder: Liedtke, Rainer K., Dr., c/o pharmed Holding GmbH, 82031 Grünwald b. München (DE)
(74) Vertreter: Oser, Andreas

(57) **Zusammenfassung**

Ein pharmazeutisches Mittel bzw. Verfahren zur selektiven Verabreichung zentralnervös therapeutisch aktiver Stoffe an spezifische Strukturen des Zentralen Nervensystems, insbesondere zur Pharmakotherapie zentralnervös bedingter Störungen und Erkrankungen wie affektiven Störungen, Depressionen und Distress, wird beschrieben.
Die zentral aktiven Stoffe werden in solche Formulierungen eingebracht mit denen sie pharmazeutisch einsetzbare Aerodispersionen bilden und mit denen sie an Chemorezeptoren der Regio Olfactoria transportiert werden. Sie induzieren dort chemisch eine Transduktion zu neuronal vermittelten Signalen an spezifische Strukturen des Zentralnervensystems. Das Verfahren ermöglicht für verschiedene zentralnervös therapeutisch aktive Stoffe chemisch-synthetischen und natürlichen endogenen Ursprungs ein neue und sichere Anwendung.

## Beschreibung

Die Erfindung betrifft ein pharmazeutisches Mittel bzw. Verfahren zur selektiven Verabreichung zentralnervös aktiver therapeutischer Stoffe in das Zentralnervensystem, insbesondere zur Verbesserung der Verträglichkeit und Effektivität von zentralnervös aktiven Stoffen bei der Therapie zentralnervös bedingter Störungen und Erkrankungen.

Es ist bekannt, daß zentralnervöse mentale Störungen, einschließlich der Symptome verschiedener Formen depressiver Verstimmung und des Distress zu den führenden medizinischen Problemen gehören bei denen Patienten ärztlichen Rat suchen. Die pharmazeutische Therapie dieser Störungen und Symptome kann formal den zentral aktiven psychopharmakologischen Arzneimitteln, im Falle depressiver Stimmung, spezifisch der Gruppe von Antidepressiva zugeordnet werden. Es ist weiterhin bekannt, daß diese Therapien in einer systemischen Weise verabreicht werden, obgleich ihr Therapieziel in Strukturen des Zentralen Nervensystems lokalisiert ist.

Der allgemeine Mechanismus mit dem zentralnervös aktive Stoffe ihre therapeutischen Effekte bei psychiatrischen Erkrankungen entfalten ist üblicherweise unbekannt, wohingegen bekannt ist, daß praktisch alle zentralnervös aktiven Arzneistoffe Effekte durch Modifikation synaptischer Transmissionen von zentralen Transmitter Substanzen erzeugen.

Psychopharmakologische Arzneimittel, üblicherweise synthetisch-chemische Stoffe, beinhalten eine substanzielle Anzahl ernster unerwünschter Nebenwirkungen. Eine wesentliche Ursache hierzu ist deren systemische Verabreichung. Eine systemische Verabreichung, zum Beispiel oral oder durch Injektion, beinhaltet die Verteilung des Stoffes im gesamten Körper und nur ein kleiner Anteil der verabreichten Substanz erreicht dabei letztlich auch sein Ziel im Zentralen Nervensystem. Folglich sind solche Effekte nicht gut kontrolliert. Zusätzlich ist bekannt, daß der therapeutische Effekt solch zentralnervöser Therapie erst nach zwei oder mehr Wochen nach Beginn der Verabreichung erkennbar wird. Verschiedene zentralnervös aktive Stoffe, insbesondere Peptide und Proteine, erreichen ihre zentralen Ziele nicht ausreichend, da sogenannte Blut-Hirn und eine Blut-Liquor-Barrieren für diese praktisch undurchlässig sind (Karzel K., Liedtke R.K., Einführung in die Arzneimitteltherapie, 2^{nd}. Edition 1985, G. Fischer, Stuttgart-New York, pp. 126-129). Die Existenz dieser biologisch wirksamen Hirn-Barrieren vermag auch zu erklären, daß solche Stoffe nach einer intrathekalen Verabreichung verglichen mit ihrer systemischen Verabreichung unterschiedliche Effekte zeigen.

Daher ist eine Therapie für zentralnervös aktive Stoffe erforderlich, die sowohl einen direkteren wie auch auch selektiveren Einfluß auf die bei mentalen Störungen involvierten Strukturen des Zentralen Nervensystems ermöglicht um sicherer und effektiver zu werden.

Derzeit wäre ein Beispiel für eine direkte Verabreichung in das Zentrale Nervensystem die intracerebrale Injektion, wie die intrathekale Injektion, welche die Hirnbarrieren überwindet und auch eine signifikante systemische Verteilung vermeidet. Aber diese Art der Verabreichung ist schwierig zu handhaben, gefährlich und schmerzhaft. Daher ist diese Methode für einen allgemeinen Gebrauch in der Therapie prohibitiv und bleibt für den erfahrenen Spezialisten bei besonderen klinischen Indikationen reserviert.

Ein basaler Aspekt für eine neue Therapie besteht daher darin, solch eine Arzneiverabreichung zu ermitteln, die ihre Effekte auf jene spezifischen Strukturen des Zentralen Nervensystems ausübt, die bei den mentalen Störungen involviert sind. Schon von der Tatsache her, daß die gegenwärtige Pharmakotherapie die systemische Verabreichung zentralnervös aktiver Stoffe nicht ändern konnte wurde es offensichtlich, daß eine praktische Umsetzung dieser Aufgabe schwierig ist und als Vorbedingung notwendig sein würde eine detaillierte Bewertung all jener biochemischen, physiologischen, anatomischen und pharmakologischen Faktoren vorzunehmen, die eine Wechselbeziehung mit mentalen Prozessen haben, bevor solch eine neue technische Methode entwickelt werden könnte. Daher sollen einige diesbezügliche Aspekte in Umrissen beschrieben werden die letztlich zu der Erfindung führten.

Ein Verarbeitungszentrum des Zentralen Nervensystems für Emotionen ist das sogenannte Limbische System mit seiner Kernregion dem Hypothalamus, der verschiedene vegetative Kerne und neurale Projektionen kontrolliert. Da die hypothalamische Region in tiefen Lagen des Mittelhirns eingebettet ist, würde.es aber nicht möglich sein, diese Struktur mit Mitteln konventioneller pharmakologischer Verabreichung zu erreichen. Andererseits verfügt dieses Zentrum, das technisch eine gewisse Rolle als "interface" für neurale und hormonelle Koordination darstellt, über eine Anzahl neuraler und hormoneller Projektionen die für pharmazeutische Zwecke besser erreicht werden könnten. Im Rahmen einer detaillierten Untersuchung und Analyse der Effekte von Neuropeptiden, die natürlicherweise in diesen Strukturen des Zentralen Nervensystems vorkommen, wurde es dann augenscheinlich, daß eine neue therapeutische Methode nur entwickelt werden könnte, die auf einer Nutzung der neuronal Verbindungen beruhte, um solche spezifisch therapeutischen Effekte zentralnervös aktiver Stoffe auf diese Struktur im Zentralen Nervensystems zu erreichen. Hierbei erschien eine Struktur, die noch nicht in der praktischen humanen Arzneitherapie mit zentralnervös aktiven therapeutischen Stoffen eingesetzt wurde vielversprechend, die sogenannte Regio Olfactoria des Riechsinnesapparates. Dies ist ein anatomisch demarkiertes kleine Gebiet mit der Fläche von 5 bis 10 cm², die in einer anatomisch verborgenen Region lokalisiert ist, im oberen hinteren Bereich über der dritten nasalen Muschel. Die äußere Oberfläche dieses kleinen Pflasters sind primäre Sinneszellen, die ein Chemorezeptor-Feld repräsentieren. Anatomisch sind diese Sinneszellen mit ihren afferenten Axonen synaptisch mit einem Netz von Dendriten der sogenannten Mitral-Zellen verbunden. Die letzteren bilden, zusammen mit einigen anderen neuronalen Zell-Typen, ein Netzwerk neural synaptischer Schaltkreise, das Gebiet des Bulbus Olfactorius. Diese Struktur ist bereits ein vorgelagerter Teil des Zentralen Nervensystems und der Bulbus projiziert, über seine olfaktorischen Nervenaxone zum Hypothalamus und dem Limbischen Cortex, somit jenen Strukturen des Zentralen Nervensystems, die in die Koordination emotionaler und vegetativer Ereignisse zentralnervös involviert sind und die Rolle eines "emotionalen Gehirns " spielen.

Dieses sensorische Pflaster ermöglicht daher, infolge seiner Funktion als Chemorezeptor, für zentralnervös aktive therapeutische Arzneimittel einen definierten Ansatz ihre molekulare chemische Information in ein direktes neuronales "hypothalamisches signallisieren" zum Zentralen Nervensystem umzuwandeln. Der Chemorezeptor kann prinzipiell, abhängig von der Rezeptorbindung des Stoffes, die von der chemischen Struktur des Stoffes abhängig ist, die chemische Stoffinformation in einen biochemischen Prozeß, der sogenannten chemischen Transduktion in bioelektrische Aktionspotentiale umwandeln. Dieser Vorgang beinhaltet auch solche Stoffe, die nicht notwendigerweise eine "riechende Komponente" enthalten, sondern auch von einem sensorischen Standpunkt eine "stille" oder chemisch verborgene Riechqualität haben können. Unterschiede in solchen sensorischen Qualitäten erscheinen schon bei nur geringfügigen chemischen Molekül-Modifikationen.

Basierend auf der Struktur der neuralen Netzwerk Verbindungen, besteht somit eine hohe Wahrscheinlichkeit, daß diese Route als Schiene für zentralnervös Effekte von zentralnervös aktiven Arzneimitteln beim Menschen genutzt werden kann. Es existieren vom Bulbus Olfactorius monosynaptische Projektionen zu supraoptischen Neuronen (Hatton, G.J. et al., Progr. Brain Res. 1998 (119) 77), somit an dem Ort, an dem zum Beispiel das hypothalamische Neuropeptid Oxytocin rasches Einsetzen von mütterlichem Verhalten bei Ratten bewirkt (Yu G.Z et al. Neuroscience 1996 72(4) 1083) und bei dem vermutlich auch das Noradrenalin System mit involviert ist (Dluzen, D.E. et al., Neurosci. Lett., 1998 254 (3) 161). Umgekehrt sollten dann aber auch negative mentale Effekte bei Veränderung dieser Bulbus Region auftreten können. Tatsächlich scheint die experimentelle Entfernung dieser Region, die Bulbektomie, ein Modell für eine Depression bei Ratten. Die operierten Tiere zeigen Veränderungen die ähnlich mit denen sind, die man bei depressiven Patienten beobachtet. Dies beinhaltet sowohl Verhaltensänderungen wie auch biochemische Änderungen, z.B. bei Noradrenalin, Serotonin, Corticosteron etc. (Kelly J.P., Pharmacol. Ther. 1997; 74 (3) 299), und die Operation potenziert auch als Streßfaktor eine hypophyseale Ausschüttung von ACTH und Corticosteron, ebenso von Vasopressin. (Marchilhac A., Horm. Metab. Res. 1999; 31 (7) 399, und Neuroscience Letter 1999; 262 (2) 89). Die Wechselbeziehungen bei Stimmungsveränderungen, depressive Stimmung und Distress, sollen dabei detaillierter für diese spezifisch zentralnervöse Route der Verabreichung am Beispiel des endogenen Hormons Oxytocin, beschrieben werden. Dies veranschaulicht auch besser die basalen Unterschiede zu den anderen technischen Routen der Verabreichung. Dieses Neuropeptid wird im Hypothalamus synthetisiert und hat teils die Rolle eines Neurotransmitters. Es ist bekannt, daß Oxytocin muskelkontrahierend auf die glatte Muskulatur des Uterus und der Milchdrüsen wirkt. Die traditionelle Sichtweise fokussierte sich nur auf periphere Oxytocin Spiegel. Daher ist die gegenwärtige klinische Anwendung begrenzt auf die Effekte am schwangeren Myometrium und myoepitheliale Zellen der laktierenden Brust. Wie bei neuen Tierforschungen gezeigt, die meist auf direkt zentralnervösen Verabreichungen (intrathecal) beruht, gehen die Rolle und pharmakologischen Effekte von Oxytocin über dies hinaus.
Tierstudien zeigten, daß Oxytocin weibliches Reproduktionsverhalten induziert, einschließlich sexueller Gefühlswallungen, Orgasmus, Gametentransport und mütterliches Verhalten, wie Brustfütterung und Bindungsverhalten zwischen Mutter und Kind, was wiederum durch Oxytocin-Antagonisten aufgehoben werden kann. (Benelli A. et al., Neuropeptides 1994,27(4) 245; Caldwell J.D. et al., Brain Res. 1990 (512) 291; Arletti R. et al. Neuropeptides 1985, 6 (3) 247). Es ist auch anerkannt, daß Oxytocin allgemein in menschliche sexuelle Gefühle involviert ist und dies mit Oxytocin Spiegeln korrespondiert (Camichael M.S et al., J. Clin.Endocrinol. Metab. 1987, 64 (1) 27; (Blaicher W. et al., Gynecol. Obstet. Invest. 1999, 47 (2) 126). Aber infolge der Nichtexistenz einer geeigneten Methode der zentralnervösen Verabreichung von Oxytocin beim Menschen ist auch über Effekte solch zentraler Verabreichung von Oxytocin beim Menschen so gut wie nichts bekannt.

Aber von seinem hypothalamischen Ursprung, insbesondere dem Nucleus paraventricularis sowie dem Nucleus supraopticus, vermag Oxytocin auch gerade die Neuronen zu sensitivieren, die mit den respektiven peripheren Muskelkontraktionen bei diesen Bedingungen verbunden sind und ein neurales feedback aktiviert, vermutlich im hypothalamischen Nucleus ventromedialis (Kow L.M et al., Neuroendocrinology 1991, 54 (5) 526), Axone zu höheren Hirnrinden Projektionen, die für Stimmung und kognitives Fühlen verantwortlich sind. Eine sensorische periphere Information, die von einem Berührungsstimulus am Penis der Ratte entsteht, erregt zum Beispiel direkt Oxytocin Zellen im Nucleus supraopticus (Honda K. et al., Brain Res. Bull., 1999, 48(3) 309).

Im Rahmen der Hormone ist es anerkannt, daß Gestresste und Depressive eine reduzierte sexuelle Aktivität zeigen und oft erhöhte Blutspiegel an Cortisol aufweisen. Zentral verabreichtes Oxytocin bewirkt hier eine dosisabhängige Reduktion des das Cortisol freisetzenden Hormons ACTH (Legros J.J. et al., J. Clin. Endocrinol Metab.. 1984, 58 (1) 105), was somit das Stresshormon Cortisol reduziert. Zentralnervöse Oxytocin Verabreichung reduzierte auch stress-induziertes Angstverhalten und zeigte in zwei Tiermodellen antidepressive Effekte, die vergleichbar waren, mit denen des tricyclischen Antidepressivums Imipramin (Arletti R. et al., Life Sci. 1987, 41 (14) 1725).
Folglich mögen die neurohormonellen Symptome von Distress und Depression auf einer Veränderung der zentralnervösen Aktivität der primär involvierten zentralnervös aktiven Stoffe gegenüber der Aktivität unter physiologischen Bedingungen sein, ein Effekt der auch Konsequenzen für die im Zentralen Nervensystem involvierten Neurotransmitter-Systeme hat, z.B. die von Noraderanline, Serotonin, GABA etc.

Eine hypothalamische Intervention mit einem wirksamen psychopharmakologischen Stoff sollte daher zu korrigierenden und gegenbalancierenden Effekten führen. Der basale Aspekt dies praktisch zu realisieren, bestand daher darin, solch eine spezifische Arzneimittelanwendung einzusetzen, die die Effekte auf diese spezifischen Strukturen des Zentralen Nervensystems initiiert. Wie vorangehend beschrieben ist somit das therapeutische Ziel die hypothalamische Region und der komplementäre Teil hierzu sind die Chemorezeptoren der Regio Olfactoria, die ihrerseits über den Bulbus Olfactorius mit dem Hypothalamus neural verbunden sind.

Daher war im nächsten Schritt die Frage zu lösen, wie dieses System therapeutisch angegangen werden kann. Der einzige Teil der in diesem System direkten Zugang für eine chemische Arzneimittelreaktion bietet, ist die Regio Olfactoria. Die Parameter für eine praktikable technische Arneiverabreichung sind daher durch die Bedingungen und Parameter dieser spezifischen Region determiniert. Sie repräsentiert für die verabreichten therapeutischen Agentien einerseits technisch die Qualität sensorischer Chemorezeptoren sowie andererseits eine anatomisch kleine wie auch verborgene Lokalisation, die nur über Luftkanäle erreicht werden kann. Eine Applikationsform, mit der ein therapeutisch zentralnervös aktiver Stoff diese Region erreichen kann erfordert daher eine geeignete flüchtige Formulierung, die das Arzneimittel ausreichend zu diesem spezifischen Rezeptor transportiert. Ebenso erfordert es, daß die therapeutischen Agentien selbst auch in solch einer Weise verarbeitet werden können, daß sie in solch einer Formulierung eingesetzt werden können.

Der Erfindung liegt die Aufgabe zugrunde, eine Verbesserung der Verträglichkeit und Effektivität von zentralnervös aktiven Stoffen bei der Therapie zentralnervös bedingter Störungen und Erkrankungen zu erreichen.

Diese Aufgabe wird dadurch gelöst, daß die im Zentralnervensystem therapeutisch aktiven Stoffe in solche Trägerformulierungen eingebracht werden mit denen sie pharmazeutisch einsetzbare Aerodispersionen bilden, die zentralnervös aktiven Stoffe dabei chemisch-synthetischen oder natürlichen Ursprungs sind und in der gebildeten Trägerformulierung in fester, flüssiger oder einer gemischten Form vorliegen, die pharmazeutische Trägerformulierung so beschaffen ist, daß sie die enthaltenen Stoffe in solcher Dosis an die Chemorezeptoren der Regio Olfactoria transportiert mit der die Chemorezeptoren chemisch zur Auslösung von Aktionspotentialen zum zentralen Nervensystem induziert werden, wobei in der Trägerformulierung einer oder mehrere der zentralnervös aktiven Stoffe enthalten sein können.

In einer weiteren Ausbildung der Erfindung sind , um den praktischen Therapieeinsatz zu erweitern, alle Stoffe einbezogen die vom klinischen Einsatz her klassifiziert sind als Neuroleptika, Tranquilizer, Thymoleptika, Thymeretika, Stimulantien, Antipsychotika, Antiepileptika oder zentrale Muskelrelaxantien,

In einer weiteren Ausbildung der Erfindung sind , um den praktischen Therapieeinsatz zu erweitern alle Stoffe einbezogen, die zentralnervös zentral aktiv sind und die therapeutisch bei der Behandlung von mentalem Stress, depressiven Befindlichkeiten, affektiven Störungen, sexuellen Dysfunktionen oder eingesetzt werden, wobei die Stoffe einzeln oder in Form von Kombinationen verabreicht werden.

In einer weiteren Ausbildung der Erfindung sind , um den praktischen Therapieeinsatz zu erweitern, alle Stoffe einbezogen, die zentralnervös aktiv sind und die therapeutisch bei zentralnervös bedingten schmerzhaften Störungen als zentralnervös wirkende Analgetika eingesetzt werden, wobei die Stoffe einzeln oder in Form von Kombinationen verabreicht werden.

In einer weiteren Ausbildung der Erfindung sind , um den praktischen Therapieeinsatz zu erweitern, alle Stoffe einbezogen die zentralnervös aktiv sind und die therapeutisch bei zentralnervös bedingten kardiovaskulären Störungen eingesetzt werden, insbesondere zentralnervös aktiven Antihypertensiva, wobei die Stoffe einzeln oder in Form von Kombinationen verabreicht werden.

In einer weiteren Ausbildung der Erfindung werden, um den praktischen Therapieeinsatz zu erweitern, alle Stoffe einbezogen, die vom pharmakologischen Mechanismus her klassifiziert sind als Monoaminoxidase-Inhibitoren, cyklische Antidepressiva, Serotonin-Reuptake Inhibitoren, Noradrenaline Reuptake Inhibitoren und dergleichen, wobei die Stoffe einzeln oder in Form von Kombinationen verabreicht werden.

In einer weiteren Ausbildung der Erfindung werden, um den praktischen Therapieeinsatz zu erweitern, alle synthetisch chemischen Stoffe einbezogen, die zentralnervös aktiv sind und die chemisch klassifiziert sind als Phenothioazine, Azaphenothiazine, Rauwolfia Alkaloide, Thioxanthene, Butyrophenone, Glykole, Diphenylmethane, Dibenzodiazepine, Karbinole, Dibenzobicyclooctadiene, Dibenzazepine, Iminodibenzyline, Iminostilbene, Dibenzocycloheptadiene und -triene, Dihydroanthracene, Acridane, Dibenzoxepine, Dibenzothiepine, Indole, Phenylethyamine, bi-, tri- und polycyclische Derivate gehören, mit allen Derivaten oder Analogen, einschließlich aller Salze, weiterhin Lithium Salze, wobei die Stoffe einzeln oder in Form von Kombinationen, verabreicht werden.

In einer weiteren Ausbildung der Erfindung sind , um den praktischen Therapieeinsatz zu erweitern, die Anwendung, die spezifischen Arzneimittel Fluoxetin, Fluvoxamin, Mirtazapin, Nefazodon, Paroxetin, Meclobemid, Reboxetin, Sertralin, Venlafaxin, Bupronion, Citalopram, in allen chemischen Formen, Analogen und Derivaten einschließlich aller Salze, enthalten, wobei die Stoffe einzeln oder in Form von Kombinationen verabreicht werden.

In einer weiteren Ausbildung der Erfindung sind, um den praktischen Therapieeinsatz zu erweitern, alle Stoffe eingesetzt, die zentralnervös aktiv sind und chemisch Peptide oder Proteine oder deren Derivate sind.

In einer weiteren Ausbildung der Erfindung sind, um den praktischen Therapieseinsatz zu erweitern, alle Stoffe eingesetzt die zentralnervös aktiv sind und als Nucleoside oder Nucleotide oder deren Derivate sind.

In einer weiteren Ausbildung der Erfindung sind, um den praktischen Therapieseinsatz zu erweitern, alle Stoffe eingesetzt die Aminosäuren und Aminosäurederivate sind oder als biogene Amine zentralnervös als Neurotansmitter wirken, inbesondere Acetylcholin, DOPA, Dopamin, Noradrenalin, 5-Hydroxytryptamin (Serotonin), Glutaminsäure, Glycin.

In einer weiteren Ausbildung der Erfindung sind , um den praktischen Therapieeinsatz zu erweitern, alle Stoffe eingesetzt, die in hypothalamische Regulationsprozesse als Neurotransmitter oder als Hormone involviert sind, einschließlich aller ihren natürlichen oder chemisch-synthetischen Analoga, Derivative und ihrer Antagonisten, einschließlich Oxytocin, Vasopressin, Met- und Leu-Enkephalin, STH, Melanoliberin, Prolactoliberin, Thyroliberin, CRH, FSH, LSH, Somatostatin, Melanostatin, Prolactostatin, wobei die Stoffe einzeln oder in Form von Kombinationen verabreicht werden.

In einer weiteren Ausbildung der Erfindung ist, um den praktischen Therapieeinsatz der neuen Methode zu erweitern, die Therapie aller depressiven Zustände, Distress und sexueller Dysfunktionen einbezogen, die durch Stoffe die über die Hypophysen-Nebennieren Achse wirken determiniert werden, insbesondere durch CRF, CRH, ACTH, Cortisol, Cortison, Adrenalin, Noradrenalin.

In einer weiteren Ausbildung der Erfindung ist, um den praktischen Therapieeinsatz zu erweitern, die Therapie aller depressiven Zustände, Distress und sexueller Dysfunktionen einbezogen, die durch Östrogene, Gestagene, Androgene, Gonadotropine, Prostaglandine determiniert werden.

In einer weiteren Ausbildung der Erfindung liegen, um den praktischen Therapieeinsatz zu erweitern, die zentralnervös aktiven Stoffe in Form von Mikro-Partikeln oder Mikro-Tropfen in der pharmazeuischen Trägerformulierung vor, bei denen die Durchmesser in einem Bereich zwischen 0,1 µm und 30 µm liegen.

In einer weiteren Ausbildung der Erfindung werden, um den praktischen Therapieeinsatz zu erweitern, für die eingesetzten zentralnervös aktiven Stoffe als pharmazeutische Lösungsmittel äthanolische Lösungen oder ätherische Öle eingesetzt.

In einer weiteren Ausbildung der Erfindung sind, um den praktischen Therapieeinsatz zu erweitern, als Anwendungshilfen Inhaliergeräte, Vernebler, Sprays oder Druckaerosole eingesetzt, die ihrerseits auch über zusätzliche mechanische Vorrichtungen zur gezielten Anwendung verfügen können, wobei eine solche Vorrichtung die Verwendung flexibler und spezifisch dimensionierter Auslaßröhren ist.

In einer weiteren Ausbildung der Erfindung wird, um den praktischen Therapieeinsatz zu erweitern, die neue Methode auch therapeutisch bei Tieren eingesetzt.

Eine selektive Verabreichung von zentral aktiven Stoffen, wie pyschopharmakologischen Therapeutika, zur hypothalamischen Region zwecks Behandlung zentralnervöser Störungen und Erkrankungen, ist bisher nicht beschrieben noch eingesetzt worden. Das Verfahren eines direkten neuronalen signalisierens zu Strukturen des Zentralen Nervensystems ist grundsätzlich unterschiedlich zur systemischen Applikation, bei der das Ziel eine Verteilung des Arzneimittels in den Kreislauf ist und aus dem hiernach das Arzneimittel seinen spezifischen Rezeptor zu finden sucht. Im neuronalen Signalprozeß wird demgegenüber die zentralnervös aktive Substanz nur bis zu dem Ziel-Chemorezeptor an der Körperoberfläche transportiert, wo dann, nach der Stoff-Rezeptor-Bindung, die chemische Information des Moleküls mittels einer biochemischen Transduktion in ein elektrisches Aktionspotential umgewandelt wird und die Aktionspotentiale spezifisch zur hypothalamischen Region weitergeleitet werden. Daher nutzt die Erfindung zur Effektauslösung eine definierte initiale Stoff-Chemorezeptor-Zone als ein therapeutisches Fenster zu spezifischen Strukturen des Zentralen Nervensystems, wobei der therapeutische Effekt neural direkt auf eine zentralnervöse Struktur projiziert wird.

Im Gegensatz dazu arbeitet die konventionelle systemische Applikation nur indirekt. Das neurale Vorgehen löst auch ein weiteres Problem der systemischen Applikationen, das der Blut-Hirn Barriere, und verhindert damit auch die mit der Verteilung verbundenen unerwünschten Nebenwirkungen. Hierüber löst die Erfindung auch spezifisch das Problem der Blut-Hirn Barriere für natürliche oder synthetische Peptid-Arzneimittel und öffnet auch diesen eine, bisher nicht bestehende, nicht-invasive Anwendung in der zentralnervösen Therapie,.
Ein weiterer Vorteil ist der kurze und direkte Weg, mit dem der Mechanismus das chemisch molekulare Bild in neurale Aktivität umsetzt. Dies erzeugt einen schnelleren Wirkeintritt. Es ist bekannt, daß sich nach systemischer Applikation zentralnervöser Therapien Effekte oft erst nach zwei oder mehr Wochen einstellen. Letztlich bewirkt das neue Verfahren auch eine Dosisverringerung.

Aus topographisch-anatomischen Gründen müssen die flüchtigen Arzneiformulierungen initial auch den Bereich der Nasenhöhle passieren um hiernach ihr Chemorezeptor-Feld zu erreichen. Die Route hat aber nichts mit einer "nasalen Verabreichung" zu tun. Letzteres meint abweichend von dieser Anwendungsform daß ein Agens auf ein nasales Schleimhaut Epithel verabreicht wird um dort eine Steigerung der Resorption in den Kreislauf zu erreichen und ist daher nur eine der konventionellen systemischen Applikationen wie z.B. buccale, sublinguale oder rektale Verabreichung. Die beschriebenen neuralen Effekte können daher auch nicht mit solcher Verabreichung eines Agens auf funktionell unspezifische Nasenschleimhaut erzielt werden.

Ein technisches Beispiel_für eine geeignet einbeziehbare pharmazeutische Formulierung für dieses neue Verfahren sind Aerodispersionen. Aerodispersionen beziehungsweise Arzneiformen mit Gasen stellen in Gasen schwebende Teilchen fester, flüssiger oder gemischter Zusammensetzung, dar, somit ein Zweiphasensystem bei dem die Stoffe in einem Gas im Zustand kolloidaler Verteilung vorliegen und bei dem diese in Form z.B. versprühbarer Zubereitungen, z.B. als Dosier-Aerosole, Sprays oder Vernebler eingesetzt werden.
Distanz und enge Dimensionen der spezifischen Luftkanäle, die von der Trägerformulierung passiert werden müssen, ohne daß es zu signifikanten vorzeitigem Niederschlag der enthaltenen Stoffe kommt, um den anatomisch verborgenen Chemorezeptor zu erreichen, stellen Bedingungen dar, die mit denen pulmonaler

Verabreichungen vergleichbar sind. Daher sind geeignete Vorrichtungen auch vergleichbar mit denen zur Lungen Inhalation, z.B. solche wie dosierte Inhalatoren, die bei Asthma eingesetzt werden. Die zentralnervös wirksamen Stoffe können für ihre Abgabe beispielsweise in einer verflüssigten Treibgasmischung gelöst werden. Im Gegensatz zu wäßrigen Aerosolen, wo nur ca. 2% des Aerosols durch Arzneistoff repräsentiert wird, können Trockenpuder Aerosole weit über 50% des Arzneistoffes enthalten. Obgleich die Dosis nicht das kritische Thema ist, da für dies neue Verfahren die Dosis gegenüber systemischer Anwendung erheblich reduziert werden kann, ergibt sich ein Vorteil solcher Trockenpuder-Arosole für die Anwendung von Peptiden. Trockenpuder Arosole können Degradationsprobleme bei Peptiden umgehen, da Peptide und Proteine im festen Zustand stabiler sind.
Da der Partikel Durchmesser bestimmt in welcher Region sich ein Partikel niederschlägt ist dieser kritischer. Um dies an der pulmonalen Verabreichung zu demonstrieren: Partikel mit einem Durchmesser um 30 µm schlagen sich bereits in der Luftröhre nieder, Partikel mit kleinerem Durchmesser von 10 µm erreichen die terminalen Bronchiolen, mit 3 um den Bereich des Ductus alveolis und mit ca. 1 µm die terminalen Lungenbläschen. Partikel mit einer Größe um 0,1 µm schlagen sich im allgemeinen in der Lunge nur noch teilweise nieder und können dort mit der Ausatemluft wieder abgeatmet werden. Im Falle dieses neuen Verfahrens sind die Partikel Durchmesser noch am ehesten vergleichbar mit den topographischen Bedingungen für eine terminale pulmonale Applikation. Allerdings sind die topographischen Bedingungen für die Luftströmungsmechanik unterschiedlich. Die anatomischen und physiologischen Bedingungen müssen präferentiell für Bereiche von 0,1 µm bis 30 µm empirisch ermittelt werden.
Eine geeignete Partikelgröße kann mit verschiedenen Methoden erreicht werden, z.B. unter anderem mittels Prozessen der Stoff-Mikronisierung, ein dem pharmazeutischen Fachmann vertrautes Verfahren. Eine Anzahl von Techniken wurde für die Präparation von Peptid- und Protein-Partikeln zum Erreichen geeigneter Größen speziell für respiratorische Arzneimittelfreisetzungen beschrieben (Johnson K.A., Advanced Drug Delivery Rev. 1997 (26) 3-15). Die Dispersion eines Arzneistoffes im Gas oder einer Flüssigkeit kann prinzipiell durch einen gasgetriebenen Aerosolgenerator erzeugt werden, dessen Druck üblicherweise im Bereich von 2 bis 6 kp/cm² liegt. Die Zerteilung erfolgt durch das spontan verdampfende Treibmittel, wobei die Partikelgröße von der Art des Wirkstoffes und des Treibmittels, dem Mengenverhältnis von Wirkstoff zu Treibmittel, sowie von technischen Eigenschaften des Ventils und des Sprühkopfes bestimmt wird.

Ein veranschaulichendes Beispiel im Falle einer Anwendung des Peptids Oxytocin, ohne es auf dieses Beispiel zu begrenzen, ist ein Aerosol bei dem die Konzentration für Oxytocin in einer flüssigen Phase im Bereich von 10-40 IU/ml liegt. Eine Einzeldosis von 0,5 IU bis 4,0 IU wird mit einem Druckstoß in Volumina von 0,05 bis 0,2 ml und Mikrotropfen in einem Bereich von 0,1 µm bis 30 µm freigesetzt.

Ein pharmazeutisches Mittel bzw. Verfahren zur selektiven Verabreichung zentralnervös therapeutisch aktiver Stoffe an spezifische Strukturen des Zentralen Nervensystems, insbesondere zur Pharmakotherapie zentralnervös bedingter Störungen und Erkrankungen wie affektiven Störungen, Depressionen und Distress, wird beschrieben. Die zentral aktiven Stoffe werden in solche Formulierungen eingebracht mit denen sie pharmazeutisch einsetzbare Aerodispersionen bilden und mit denen sie an Chemorezeptoren der Regio Olfactoria transportiert werden. Sie induzieren dort chemisch eine Transduktion zu neuronal vermittelten Signalen an spezifische Strukturen des Zentralnervensystems. Das Verfahren ermöglicht für verschiedene zentralnervös therapeutisch aktive Stoffe chemisch-synthetischen und natürlichen endogenen Ursprungs ein neue und sichere Anwendung.

## Patentansprüche

1. Pharmazeutisches Verfahren zur selektiven Verabreichung zentralnervös aktiver therapeutischer Stoffe in das Zentralnervensystem, dadurch kennzeichnet, daß im Zentralnervensystem therapeutisch aktive Stoffe in solche Trägerformulierungen eingebracht werden mit denen sie pharmazeutisch einsetzbare Aerodispersionen bilden, die zentralnervös aktiven Stoffe dabei chemisch-synthetischen oder natürlichen Ursprungs sind und in der gebildeten Trägerformulierung in fester, flüssiger oder einer gemischten Form vorliegen, die pharmazeutische Trägerformulierung so beschaffen ist, daß sie die enthaltenen Stoffe in solcher Dosis an die Chemorezeptoren der Regio Olfactoria transportiert mit der die Chemorezeptoren chemisch zur Auslösung von Aktionspotentialen zum zentralen Nervensystem induziert werden, wobei in der Trägerformulierung einer oder mehrere der zentralnervös aktiven Stoffe enthalten sein können.

2. Pharmazeutisches Verfahren nach Anspruch 1, **dadurch gekennzeichnet**, daß alle Stoffe einbezogen sind die vom klinischen Einsatz her klassifiziert sind als Neuroleptika, Tranquilizer, Thymoleptika, Thymeretika, Stimulantien, Antipsychotika, Antiepileptika oder zentrale Muskelrelaxantien,

3. Pharmazeutisches Verfahren nach vorgehenden Ansprüchen, **dadurch gekennzeichnet**, daß alle Stoffe einbezogen sind, die zentralnervös aktiv sind und therapeutisch bei der Behandlung von mentalem Stress, depressiven Befindlichkeiten, affektiven Störungen, sexuellen Dysfunktionen oder eingesetzt werden, wobei die Stoffe einzeln oder in Form von Kombinationen verabreicht werden.

4. Pharmazeutisches Verfahren nach vorgehenden Ansprüchen, **dadurch gekennzeichnet**, daß alle Stoffe einbezogen sind die zentralnervös aktiv sind und die therapeutisch bei zentralnervös bedingten schmerzhaften Störungen als zentralnervös wirkende Analgetika eingesetzt werden, wobei die Stoffe einzeln oder in Form von Kombinationen verabreicht werden.

5. Pharmazeutisches Verfahren nach vorgehenden Ansprüchen, **dadurch gekennzeichnet**, daß alle Stoffe einbezogen sind, die zentralnervös aktiv sind und die therapeutisch bei zentralnervös bedingten kardiovaskulären Störungen eingesetzt werden, insbesondere zentralnervös aktive Antihypertensiva, wobei die Stoffe einzeln oder in Form von Kombinationen verabreicht werden.

6. Pharmazeutisches Verfahren nach vorgehenden Ansprüchen, **dadurch gekennzeichnet**, daß alle Stoffe einbezogen sind, die vom pharmakologischen Mechanismus her klassifiziert sind als Monoaminoxidase-Inhibitoren, cyklische Antidepressiva, Serotonin-Reuptake Inhibitoren, Noradrenaline Reuptake Inhibitoren und dergleichen, wobei die Stoffe einzeln oder in Form von Kombinationen verabreicht werden.

7. Pharmazeutisches Verfahren nach vorgehenden Ansprüchen, **dadurch gekennzeichnet**, daß alle synthetisch chemischen Stoffe einbezogen sind , die chemisch klassifiziert sind als Phenothioazine, Azaphenothiazine, Rauwolfia Alkaloide, Thioxanthene, Butyrophenone, Glykole, Diphenylmethane, Dibenzodiazepine, Karbinole, Dibenzobicyclooctadiene, Dibenzazepine, Iminodibenzyline, Iminostilbene, Dibenzocycloheptadiene und -triene, Dihydroanthracene, Acridane, Dibenzoxepine, Dibenzothiepine, Indole, Phenylethyamine, bi-, tri- und polycyclische Derivate gehören, mit allen Derivaten oder Analogen, einschließlich aller Salze, sowie auch Lithium Salze, wobei die Stoffe einzeln oder in Form von Kombinationen, verabreicht werden.

8. Pharmazeutisches Verfahren nach vorgehenden Ansprüchen, **dadurch gekennzeichnet**, daß die Anwendung, der spezifischen Arzneimittel Fluoxetin, Fluvoxamin, Mirtazapin, Nefazodon, Paroxetin, Meclobemid, Reboxetin, Sertralin, Venlafaxin, Bupronion, Citalopram, in allen chemischen Formeln, Analogen und Derivaten einschließlich aller Salze, einbezogen ist, wobei die Stoffe einzeln oder in Form von Kombinationen verabreicht werden.

9. Pharmazeutisches Verfahren nach vorgehenden Anspiüchen, **dadurch gekennzeichnet**, daß alle Stoffe einbezogen sind, die zentralnervös aktiv sind und chemisch Peptide, Proteine oder deren Derivate sind wobei die Stoffe einzeln oder in Form von Kombinationen verabreicht werden.

10. Pharmazeutisches Verfahren nach vorgehenden Ansprüchen, **dadurch gekennzeichnet**, daß alle Stoffe einbezogen sind die zentralnervös aktiv und Nucleoside, Nucleotide oder deren Derivate sind wobei die Stoffe einzeln oder in Form von Kombinationen verabreicht werden.

11. Pharmazeutisches Verfahren nach vorgehenden Ansprüchen, **dadurch gekennzeichnet**, daß alle Stoffe einnezogen sind die zentralnervös aktiv und Aminosäuren oder Aminosäurederivate sind, oder die als biogene Amine zentralnervös als Neurotransmitter wirken, inbesondere Acetylcholin, DOPA, Dopamin, Gamma amino Buttersäure (GABA), Noradrenalin, 5-Hydroxytryptamin (Serotonin), Glutaminsäure, und Glycin wobei die Stoffe einzeln oder in Form von Kombinationen verabreicht werden.

12. Pharmazeutisches Verfahren nach vorgehenden Ansprüchen, **dadurch gekennzeichnet**, daß alle Stoffe einbezogen sind, die in hypothalamische Regulationsprozesse als Neurotransmitter oder als Hormone involviert sind, einschließlich aller ihren natürlichen oder chemisch-synthetischen Analoga, Derivative und ihrer Antagonisten, insbesondere Oxytocin, Vasopressin, Met- und Leu-Enkephalin, STH, Melanoliberin, Prolactoliberin, Thyroliberin, CRH, FSH, LSH, Somatostatin, Melanostatin, Prolactostatin, wobei die Stoffe einzeln oder in Form von Kombinationen verabreicht werden.

13. Pharmazeutisches Verfahren nach vorgehenden Ansprüchen, **dadurch gekennzeichnet**, daß die Therapie aller depressiven Zustände, von mentalem Distress und sexuellen Dysfunktionen einbezogen ist, die durch Stoffe determiniert werden die über die Hypophysen-Nebennieren Achse wirken, insbesondere CRF, ACTH, CRH, Cortisol, Cortison, Adrenalin, Noradrenalin.

14. Pharmazeutisches Verfahren nach vorgehenden Ansprüchen, **dadurch gekennzeichnet**, daß die Therapie aller depressiven Zustände, von mentalem Distress und sexueller Dysfunktionen einbezogen ist, die durch Östrogene, Gestagene, Androgene, Gonadotropine, Prostaglandine determiniert werden.

15. Pharmazeutisches Verfahren nach vorgehenden Ansprüchen, **dadurch gekennzeichnet**, daß die eingesetzten zentralnervös aktiven Stoffe in Form von Mikro-Partikeln oder Mikro-Tropfen in der pharmazeutischen Trägerformulierung vorliegen, bei denen die Durchmesser in einem Bereich zwischen 0,1 µm und 30 µm liegen.

16. Pharmazeutisches Verfahren nach vorgehenden Ansprüchen, **dadurch gekennzeichnet**, daß für die eingesetzten zentralnervös aktiven Stoffe als pharmazeutische Lösungsmittel äthanolische Lösungen oder ätherische Öle eingesetzt werden,

17. Pharmazeutisches Verfahren nach vorgehenden Ansprüchen, **dadurch gekennzeichnet**, daß als Anwendungshilfen Inhaliergeräte, Vernebler, Sprays oder Druckaerosole eingesetzt werden, die ihrerseits über zusätzliche mechanische Vorrichtungen zur gezielten Anwendung verfügen können, wobei eine solche Vorrichtung die Verwendung flexibler und spezifisch dimensionierter Auslaßröhren ist.

18. Pharmazeutisches Verfahren nach vorgehenden Ansprüchen, **dadurch gekennzeichnet**, daß es therapeutisch auch bei Tieren eingesetzt wird.
